# EUROPÄISCHE PATENTSCHRIFT

(11) **EP 4 344 609 B1**
(45) Veröffentlichungstag und Bekanntmachung des Hinweises auf die Patenterteilung: **01.10.2025**
(21) Anmeldenummer: 22199043.5
(22) Anmeldetag: 30.09.2022
(51) Int. Cl.: A61B 1/12

(54) **VERFAHREN UND VORRICHTUNG ZUR REINIGUNG VON MEDIZINISCHEN INSTRUMENTEN MITTELS MODULIERENDER DRUCKGASIMPULSE**
METHOD AND APPARATUS FOR CLEANING MEDICAL INSTRUMENTS USING MODULATING COMPRESSED GAS PULSES
PROCÉDÉ ET APPAREIL DE NETTOYAGE D'INSTRUMENTS MÉDICAUX AU MOYEN D'IMPULSIONS DE GAZ SOUS PRESSION MODULANTES

(43) Veröffentlichungstag der Anmeldung: 03.04.2024
(73) Patentinhaber: Hammann GmbH, 76829 Landau (DE)
(72) Erfinder: LÄMMER, Paulina, 80698 München (DE); KLEIN, Norbert, 57520 Grosbliederstroff (FR)
(74) Vertreter: Patentanwälte Dr. Keller, Schwertfeger Partnerschaft mbB

(56) Entgegenhaltungen:
- EP-A1- 2 065 059
- EP-A1- 3 646 773
- US-A1- 2011 097 248

## Beschreibung

Die vorliegende Erfindung betrifft ein Verfahren und eine Vorrichtung zur Reinigung von medizinischen Instrumenten, die wenigstens einen zu reinigenden Hohlkanal umfassen, mittels modulierender Druckgasimpulse.

In nahezu allen Fachbereichen der Human, Dental- und Veterinärmedizin werden wiederverwendbare medizinische Instrumente, die wenigstens ein Lumen oder einen Hohlkanal aufweisen, verwendet. Beispiele solcher Instrumente sind Endoskope, Gastroskope, Koloskope, Rektoskope, Proktoskope, Laparoskope, Arthroskope, Bronchoskope, Thoraskope, Sonden, Schläuche oder Katheter.

Die Untersuchungen mit medizinischen Instrumenten werden in Körperöffnungen durchgeführt, die mit Mikroorganismen besiedelt sind. Infolgedessen werden die Instrumente kontaminiert. Ein angeschmutztes Endoskop erfordert nach der Untersuchung und vor dem Desinfizieren oder dem Sterilisieren gründliches und effizientes Reinigen (als "Vorreinigen" bekannt). Zur Widerverwendung ist daraufhin eine hygienische Reinigung unerlässlich, denn ein Instrument, das nicht vollständig gesäubert wurde, stellt eine Infektionsgefahr dar.

Die Aufbereitung von flexiblen Endoskopen und anderen medizinischen Geräten ist komplex. Sie besteht meistens aus der Vorreinigung sowie der anschließenden Desinfektion und der Konditionierung einschließlich Trocknung. Während Desinfektion und Konditionierung in Vorrichtungen automatisch läuft, wird die Vorreinigung bisher manuell von medizinischem Fachpersonal durchgeführt. Dies ist nicht besonders wirtschaftlich und schwer zu validieren. Händisch durchgeführte Arbeitsschritte bedingen ein erhöhtes Risiko der Kontamination von Patient, Personal und Umgebung.

Bisher erfolgt die Vorreinigung dieser Instrumente manuell, in Deutschland entsprechend nach Leitlinie des Robert-Koch-Instituts (RKI) und des Bundesinstitutes für Arzneimittel und Medizinprodukte (BfArM) . Diese manuelle Vorreinigung wird derzeit mit vorwiegend nichtschäumenden Reinigern und Bürsten durchgeführt. Die bisherige Vorreinigung beinhaltet einen Dichtigkeitstest, äußerliches Abwischen und anschließendes Einlegen in ein Reinigungsbad. Häufig handelt es sich um ein Multienzym-Reinigungsmittelbad, wobei mitunter auch andere Reinigungsmittel anstelle von Enzymen verwendet werden. Die Reinigungsflüssigkeit wird arbeitstäglich oder nach starker Verschmutzung gewechselt. Die Arbeitskanäle eines Endoskops werden mit einer Bürste gereinigt, wobei der Austrag in die Flüssigkeit des Reinigungsbades gelangt. Anschließend wird diese Reinigungsflüssigkeit im Becken durch die Endoskopkanäle gespült. Schließlich erfolgt ein Blasen mit Luft, um die Oberflächen im Wesentlichen zu trocknen, so dass eine Verdünnung von Desinfektionsmitteln vermieden wird. Erst danach ist das Endoskop für die Desinfizierung oder Sterilisierung bereit.

Eigentlich sollten für die Vorreinigung jedes Kanals neue Bürsten verwendet werden. Aber üblicherweise werden Reinigungsbürsten, die für eine Endoskop-Reinigungsvorrichtung verwendet werden, zwischen jedem Anwendungszyklus Reinigen und Desinfizieren unterzogen, indem sie mit Wasser gespült werden, in ein Enzymbad eingeweicht werden und üblicherweise derselben Desinfektion ausgesetzt werden, wie das Endoskop. Die Anzahl der Anwendungszyklen solcher Reinigungsbürsten ist nicht bestimmt. Viele davon bleiben in Verwendung, bis Borsten weggebrochen sind und in Folge Endoskopkanäle verletzt werden. Weiterhin muss für jeden Kanaldurchmesser die richtige Bürste verwendet werden. Darüber hinaus können nicht alle Kanäle gebürstet werden, weil sie teilweise zu kleine Kanaldurchmesser haben und dafür keine Bürsten vorhanden sind.

Durch eine manuelle Vorreinigung ergibt sich ein erhebliches Kontaminationsrisiko für die Personen, die diese Prozedur durchführen. Es zeigt sich, dass die Qualität der manuellen Vorreinigung Schwankungen unterliegt, die beispielsweise durch die unterschiedlichen Anwender und Bürsten hervorgerufen werden. In Folge dessen können die medizinischen Geräte kontaminiert sein und somit eine Kontaminationsgefahr für die Patienten darstellen. Für das manuelle Reinigungsverfahren entsteht ein hoher Verbrauch an Wasser und Einmalprodukten wie beispielsweise Handschuhen und Bürsten.

Einige Eingriffe, beispielsweise Diagnoseuntersuchungen der menschlichen Speiseröhre und des Magens, können innerhalb von 10 bis 15 Minuten, nachdem der Patient sediert wurde, erledigt werden, während das Vorreinigen etwa 15 Minuten und das Desinfizieren 40 bis 50 Minuten erfordert. Dies ist unbefriedigend.

Die EP 3 646 773 A1 beschreibt ein Verfahren zur Reinigung, Aufbereitung und Prüfung von Hohlkörperinstrumenten, insbesondere Endoskopen, wobei die Reinigung über ein Durchziehen eines Reinigungswerkzeuges, beispielsweise eines Sweepers oder dergleichen durch die Kanäle des Instruments erfolgt.

Die DE 10 2004 040 734 B3 und DE 103 21 991 B3 beschreiben eine Vorrichtung zum Reinigen und/oder Desinfizieren von Hohlkörpern, insbesondere von medizinischen Schläuchen, Kathetern oder Endoskopen mit mindestens einer an einen Wasserkanal angeschlossenen Kupplung zur Verbindung des Wasserkanales mit dem Hohlkörper, wobei in Strömungsrichtung vor der Kupplung eine Strömungsverengung in Form einer einstellbaren Düse und ein Drucksensor angeordnet sind.

Die EP 0 71 1 529 A1 beschreibt ein Verfahren zum Prüfen und Reinigen von Instrumenten für die minimal invasive Chirurgie oder minimal invasive Untersuchung von Körperhöhlen. Dabei wird der Kanal auf Durchlässigkeit geprüft und bei zu geringer oder fehlender Durchströmung das betreffende Instrument als nicht ausreichend durchspülbar erkannt und zur Aussonderung registriert.

US 10,772,491 B2 beschreibt ein Verfahren zur Reinigung eines Endoskops in einem computergestützten gesteuerten Reinigungs-/Desinfektionsgerät, bei dem jedes Lumen eines Endoskops an ein Flüssigkeitsverteilungssystem angeschlossen wird, um selektiv Druckluft oder Druckflüssigkeiten durch ein Lumen in ein Endoskop zu befördern.

Die WO2013037491A1 beschreibt eine Vorrichtung zur Spülung von Endoskopkanälen eines Endoskops mit Spülmittel aus einem Spülmittelvorrat, wobei die Vorrichtung zwei Spülmittelverteiler, die jeweils mit dem Spülmittelvorrat verbundenen sind, sowie eine Anzahl von Spülkanälen zur Einleitung von Spülmittel aus dem Spülmittelvorrat in jeweils einen Endoskopkanal umfasst. Ferner ist eine Prüfvorrichtung vorgesehen, mittels der ein über einen der Spülkanäle an dem ersten Spülmittelverteiler angeschlossener Endoskopkanal auf eine Verstopfung hin überprüfbar ist.

Die DE69837790T2 beschreibt ein Verfahren zur Entfernung von Biofilm und Debris von den inneren Oberflächen eines Rohrs/Schlauchs, umfassend das Leiten eines Stroms einer Mischphasen-Reinigungszusammensetzung, wobei die Zusammensetzung eine wässerige Lösung von Wasser und einem Tensid in Mischung mit einem unter Druck stehenden gepulsten Gas umfasst, um eine turbulente Mischung oder einen Schaum zu erzeugen. Durch den Druck im Rohr oder im Schlauch wird für einen dynamischen Aufprall gesorgt, welcher den Biofilm und die Debris entfernt. Vorgesehen ist auch, dass eine Spülung von Wasser und Druckluft durch das Rohr oder den Schlauch erfolgt.

In der DE 35 02 969 A1 werden ein Verfahren und eine Vorrichtung zum Reinigen einer Rohrleitung mit Hilfe von gleichzeitig eingeleiteten Impulsen einer Flüssigkeit oder eines Gases beschrieben, wobei sich diese Impulse zu Gesamtimpulsen mischen, welche die Rohrleitung intermittierend durchsetzen. Dabei werden die Impulse der Flüssigkeit oder des Gases in einzelne Impulse zerlegt.

Die EP 2 065 059 A1 beschreibt ein Verfahren und eine Vorrichtung zur Reinigung von medizinischen Geräten, insbesondere von Endoskopen. Hierbei erfolgt eine Beaufschlagung von Luft und Wasser in einer Spülstrecke, um so das darin befindliche Wasser vor sich her zu treiben. Die Luftversorgung umfasst einen Regler für den zur Verfügung gestellten Luftdruck. Entsprechend weist auch die Wasserversorgung einen Regler für den Wasserdruck auf. Ein Impulsformer steuert ein Magnetventil an, so dass Luft mit einem höheren Druck als das an einer Rückschlagklappe anstehende Wasser durch das Magnetventil strömen kann. Eine weitere Rückschlagklappe öffnet auf Grund des höheren Luftdrucks, so dass Luft in die Leitung zwischen der Rückschlagklappe für das Wasser und der Spülstrecke eintreten kann.

Die US 2011/0097248 A1 beschreibt eine Vorrichtung zur Reinigung von Endoskopen mittels einer Flüssigkeit, die vorzugsweise Reinigungsmittel und Desinfektionsmittel enthält.

Vor diesem Hintergrund ist es daher Aufgabe der vorliegenden Erfindung, ein verbessertes Verfahren und eine Vorrichtung zur schnellen und effizienten (Vor)-Reinigung von medizinischen Instrumenten bereitzustellen, die eine voll integrierte und automatisierte Reinigung ermöglichen und durch reproduzierbare Reinigungsergebnisse in kurzer Zeit validierbar sind.

Diese Aufgabe wird gelöst durch ein Impulsspülverfahren mit den Merkmalen des Anspruchs 1 und eine entsprechende Vorrichtung zur Durchführung eines solchen Verfahrens. Bevorzugte Ausführungsvarianten finden sich in den Unteransprüchen wieder.

Das erfindungsgemäße Verfahren zur Reinigung von medizinischen Instrumenten, insbesondere Endoskopen, basiert auf einer Beaufschlagung eines mit Spülflüssigkeit teilbefüllten Hohlkanals des medizinischen Instruments mit modulierenden Druckgasimpulsen zur Ausbildung von alternierenden Flüssigkeitsblöcken und Gasblöcken, die impulsartig entlang einer Spülstrecke von einer Einspeisestelle durch den Hohlkanal zur Entfernung von Ablagerungen an den Wänden des Hohlkanals zu einer Ausspeisestelle getrieben werden. Die alternierenden Flüssigkeitsblöcke und Gasblöcke führen zu Scherkräften an den Wandungen des Hohlkanals des medizinischen Instruments, was zum mechanischen Ablösen von Ablagerungen oder Kontaminationen führt. Ein Hohlkanal im Sinne der Erfindung bezeichnet hierbei ein umseitig geschlossenes Lumen oder einen Hohlkörper bei einem medizinischen Instrument, also beispielsweise einem Endoskop.

Der zu reinigenden Hohlkanal des medizinischen Instruments wird in einem ersten Verfahrensschritt über ein oder mehrerer Kopplungselemente an eine Druckimpulseinrichtung fluiddicht angekoppelt, mit der die Druckluftimpulse erzeugt werden. Gleichzeitig ist eine gezielte Flüssigkeitszufuhr für die Bildung der Flüssigkeitsblöcke erforderlich. Vorzugsweise ist für jeden Hohlkanal des medizinischen Instruments ein eigenes Kopplungselement vorgesehen. Die Kopplungselemente können verschiedenartige Größen und Typen aufweisen, so dass unterschiedliche medizinische Instrumente verschiedener Hersteller an die Reinigungseinrichtung oder die Druckimpulseinrichtung angekoppelt werden können.

Nach der Ankopplung des Hohlkanals des medizinischen Instruments an die Kopplungselemente der Druckimpulseinrichtung erfolgt erfindungsgemäß eine Teilbefüllung und anschließend eine Beaufschlagung des Hohlkanals mit von der Druckimpulseinrichtung erzeugten modulierenden Druckgasimpulsen zur Ausbildung von alternierenden Flüssigkeitsblöcken und Gasblöcken, die impulsartig entlang einer Spülstrecke von einer Einspeisestelle durch den Hohlkanal zur Entfernung von Ablagerungen an den Wänden des Hohlkanals zu einer Ausspeisestelle getrieben werden. Bei der Spülflüssigkeit kann es sich um Wasser, Reinigungsflüssigkeit oder eine Lösung handeln, die u.a. auch Enzyme, Desinfektionsmittel oder Reinigungschemikalien umfasst.

Zwar sind solche Druckimpulsspülverfahren an sich bekannt, aber im Bereich der medizinischen Instrumente mit ihren Hohlkanälen mit geringen Nennweiten sind sie nicht einsetzbar oder wären nicht effizient genug, um den hohen hygienischen Anforderungen zu genügen.

Um die Effizienz der Reinigung zu erhöhen, wird erfindungsgemäß vor der Spülstrecke des Hohlkanals des medizinischen Instruments wenigstens ein Vorlaufkanal zur Beschleunigung des Flüssigkeitsvolumens in der Spülstrecke angeordnet. Der Vorlaufkanal dient als Beschleunigungsstrecke für die sich bildenden Flüssigkeitsblöcke. Dafür wird der Vorlaufkanal vor der Beaufschlagung mit den Druckgasimpulsen zunächst mit der Spülflüssigkeit teilbefüllt. Vorzugsweise enthält die Druckimpulseinrichtung deshalb Anschlüsse und Leitungen für die Teilbefüllung des Vorlaufkanals. Vorzugsweise reicht eine Teilbefüllung des Vorlaufkanals mit Flüssigkeit von etwa 10 bis 30 % des Leitungsvolumens aus. Vorzugsweise erfolgt die Teilbefüllung des Vorlaufkanals durch ein gezieltes Dosieren der Druckgaszufuhr und der Spülflüssigkeitszufuhr abhängig von der Impulsdauer und Intervalldauer. Diese Regelungseinrichtungen sind Bestandteil der Druckimpulseinrichtung.

Der Vorlaufkanal ist hinsichtlich seiner Leitungsgeometrie, seines Leitungsdurchmessers und/oder seiner Leitungslänge so dimensioniert, dass sich bei Beaufschlagung des Druckgasgemisches die Flüssigkeitsblöcke innerhalb des Vorlaufkanals vollständig ausbilden können, um den Leitungsquerschnitt der sich daran anschließenden Spülstrecke leitungsausfüllend zu durchwandern. Mit Hilfe des Vorlaufkanals ist es somit möglich, die Reinigung von medizinischen Instrumenten mittels Druckgasimpulsen weitaus effizienter durchzuführen. Tatsächlich zeigen die von den Erfindern gewonnenen Daten, dass die Wandschubspannung bei Vorschaltung eines Vorlaufkanals vor der eigentlichen Spülstrecke (Reinigungsabschnitt) wesentlich höher ist als ohne einen solchen Kanal. Der vor Spülstrecke oder Reinigungsabschnitt angeordnete Vorlaufkanal für die Flüssigkeitsblöcke bewirkt, dass sich die Flüssigkeitsblöcke bis zum Anfang der Spülstrecke des Hohlkanals des medizinischen Instruments leitungsausfüllend ausbilden können. Der Vorlaufkanal hat somit die Funktion einer Beschleunigungsstrecke für die sich bildenden Flüssigkeitsblöcke und stellt sicher, dass diese vor Beginn der Spülstrecke vollständig leitungsausfüllend ausgebildet sind. Die an der Kanalwandung der Spülstrecke vorhandenen Ablagerungen des Hohlkanals des medizinischen Instruments werden wegen der sich entwickelnden großen Scherkräfte und Wandschubspannungen entfernt.

Der vor der eigentlichen Spülstrecke angeordnete Vorlaufkanal bzw. die damit gewonnene Beschleunigungsstrecke ermöglicht eine effizientere Desinfektion, Konditionierung und Zustandsbewertung. Der erfindungsgemäße Vorlaufkanal zeichnet sich auch dadurch aus, dass der Energieaufwand zur Reinigung gegenüber herkömmlichen Impulsspülverfahren deutlich reduziert wird. Auch wird die Effizienz des Verfahrens durch die Anordnung des Vorlaufkanals vor der eigentlichen Spülstrecke verbessert. Das erfindungsgemäße Verfahren zeichnet sich ferner dadurch aus, dass die Reinigung der Hohlkanäle und damit z.B. die Vorreinigung von Endoskopen gegenüber den herkömmlichen Reinigungsverfahren deutlich hygienischer wird und schneller durchgeführt werden kann.

Der hier verwendete Begriff "Druckgas" umfasst in der Regel ein komprimiertes Gas oder ein Gasgemisch. Der Begriff "Gas" umfasst nicht nur reine Gase, sondern auch Gasgemische. Vorzugsweise wird in der vorliegenden Erfindung Druckluft als Druckgas eingesetzt. Jedoch sind grundsätzlich auch andere (inerte) Gase oder Gasgemische als Druckgas einsetzbar, beispielsweise Argon, Stickstoff oder Kohlendioxid. Die Höhe des Druckes beträgt vorzugsweise zwischen 3 und 10 bar.

Der hier verwendete Begriff "Spülstrecke" bezeichnet den zu reinigenden Hohlkanalabschnitt des zu reinigenden Hohlkanals des medizinischen Instruments und kann auch die Anschlüsse umfassen.

Der hier verwendete "Vorlaufkanal" beschreibt einen Hohlkanalabschnitt, der vor der eigentlichen Spülstrecke angeordnet ist.

Die Begriffe "Spülung" und "Reinigung" werden erfindungsgemäß synonym verwendet und bezeichnen entweder eine Spülung oder eine Reinigung eines Hohlkanals, wobei das eine auch das andere bedingen kann.

In bevorzugten Varianten des Impulsspülverfahrens wird vor der Beaufschlagung des Hohlkanals mit modulierenden Druckgasimpulsen ein Dichtigkeitstest durchgeführt. Dieser prüft die Dichtigkeit oder Dichtheit des Raums zwischen Außenhülle des Endoskops zu seinen Einzelkanälen. So wird sichergestellt, dass die Reinigung fehlerfrei und damit zuverlässig hygienisch erfolgen kann.

In einer weiter bevorzugten Variante wird vor der Beaufschlagung des Hohlkanals mit modulierenden Druckgasimpulsen ein Drucktest zur Prüfung der Dichtigkeit des Raums zwischen Endoskophülle und der Außenseite der innenliegenden Kanäle des medizinischen Instruments durchgeführt.

In einer weiter bevorzugten Variante des Impulsspülverfahrens ist vorgesehen, dass die Spülflüssigkeit während des Druckgasimpulses angesaugt und über eine Venturidüse in dem zu reinigenden Hohlkanal zerstäubt wird. Die Druckgasimpulse verteilen die Spülflüssigkeit an die Innenwände des zu reinigenden Geräts. Wenn die Spülflüssigkeit zusätzliche Hilfsstoffe (z.B. Detergenzien oder Enzyme) umfasst, ist die Einwirkdauer wegen der Konzentration der Hilfsstoffe in diesen Lösungen relativ kurz.

Diese Variante kann für eine Vorreinigung vor der eigentlichen Impulsspülreinigung verwendet werden. Die nach der Vorreinigung eventuell verbliebenen Restmengen an Kontaminationen werden in ihrer Struktur verändert, damit sie bei der nachfolgenden Impulsspülreinigung mobilisiert und vollständig entfernt werden können. Die nachfolgende Impulsspülreinigung sorgt für optimalen hygienisch einwandfreie Oberflächen. Die anschließend in die Spülstrecke beaufschlagten Druckgasimpulse ohne Wasserzufuhr trocknen und konditionieren die Innenwände des Hohlkanals des medizinischen Instruments.

Es ist alternativ vorgesehen, dass für die Beaufschlagung des Hohlkanals mit modulierenden Druckgasimpulsen ein Vorratsgefäß für die Spülflüssigkeit bereitgestellt wird, bei dem ein definierter Vordruck eingestellt wird. Vorzugsweise erfolgt das Dosieren der Wasser- und Druckgaszufuhr über den Vorlaufkanal abhängig von Impuls- und Intervalldauer.

Typischerweise kommt das erfindungsgemäße Impulsspülverfahren bei unterschiedlichsten medizinischen Instrumenten mit wenigstens einem Hohlkanal zum Einsatz. Vorzugsweise handelt es sich um ein Endoskop, ein Gastroskop, ein Koloskop, ein Rektoskop, ein Proktoskop, ein Laparoskop, ein Arthroskop, ein Bronchoskop, ein Thoraskop, eine Sonde, ein Röhrchen, einen Schlauch oder einen Katheter. Es können aber auch fest installierte medizinische Einrichtungen sein. Die verschiedenen Anwendungsbereiche und Hersteller führen zu unterschiedlichen Durchmessern der zu reinigenden Hohlkanäle. Vorzugsweise weisen die zu reinigende Hohlkanäle des medizinischen Instruments einen Durchmesser von 1 bis 20 mm, vorzugsweise einen Durchmesser von 1 bis 7 mm auf.

Medizinische Geräte verfügen oftmals über mehrere Hohlkanäle, die unterschiedlich stark verschmutzt sind und damit unterschiedliche Anforderungen an die Reinigung haben. Zur hygienischen Reinigung der Hohlkanäle ist in solchen Fällen eine festzulegende Einwirkzeit der Reinigungslösung notwendig. Vorzugsweise ist vor oder zwischen der Beaufschlagung des Hohlkanals mit modulierenden Druckgasimpulsen ein Einwirkintervall vorgesehen, bei dem eine Spülflüssigkeit bereitgestellt wird. Bedarfsweise werden der Spülflüssigkeit in bevorzugten Ausführungsvarianten Hilfsstoffe, Desinfektionsmittel, Enzyme oder Chemikalien beigemengt. Die Spülflüssigkeit wird in dem zu reinigenden Hohlkanal dann für eine festgelegte Zeitdauer inkubiert. In einer alternativen Variante erfolgt die Beimengung über eine separate Zuführleitung in den Vorlaufkanal. Die verschiedenen Hohlkanäle sind einzeln ansteuerbar. So ist es möglich, erst einen Hohlkanal zu reinigen, die Spülflüssigkeit hinzuzufügen und für eine ausreichende Zeitdauer inkubieren zu lassen, während in der Inkubationszeit die anderen Hohlkanäle gereinigt werden. Zur Reinigung können verschiedenartige Lösungen verwendet werden. Es können mehrere Reinigungssequenzen im Wechsel mit Inkubationszeiten erfolgen.

Bei den beigemengten Hilfsstoffen handelt es sich vorzugsweise um Stoffe zur analytischen Bestimmung von Restkontamination. Hierfür werden vorzugsweise verschiedene Biomarker eingesetzt. Die gereinigten Hohlkanäle werden in einer bevorzugten Ausführungsvariante anschließend vorzugsweise mit Druckluft getrocknet.

Eine Zudosierung von Hilfsstoffen über den Vorlaufkanal erfolgt vorzugsweise dann, wenn nach der Vorreinigung weitere Behandlungen zur Desinfektion vorgesehen sind, wie z.B. ein automatisches Zudosieren von Enzymlösungen oder Chemikalienlösungen, oder das Zudosieren von Hilfsstoffen zur analytischen Bestimmung von Restkontaminationen. Die Zuführung solcher Hilfsstoffe kann vorzugsweise über eine separate Zuführleitung erfolgen. Nach dem genauen Zudosieren von Zusatzstoffen wie z.B. Enzymlösungen oder Chemikalienlösungen kann eine Kreislaufführung zum Einsatz kommen. Dazu befindet sich an der Ausspeisestelle eine Armatur, welche die Lösung über eine langsam laufende Pumpe zum Vorlaufkanal befördert. Die Lösung wirkt auf die nach der Vorreinigung eventuell verbliebenen Restmengen an Kontaminationen ein und verändert sie in ihrer Struktur so, dass sie bei der nachfolgenden Impulsspülreinigung mobilisiert und vollständig entfernt werden können.

Das erfindungsgemäße Impulsspülverfahren ermöglicht eine automatisierte Vorreinigung und ist gegenüber der händischen Bürstenreinigung sicherer und wirtschaftlicher. Die Reinigung lässt sich zuverlässig validieren und schont darüber hinaus die teuren medizinischen Geräte.

Neben dem Verfahren betrifft die Erfindung auch eine Vorrichtung zur Reinigung von medizinischen Instrumenten mit wenigstens einem Hohlkanal mittels Beaufschlagung des Hohlkanals modulierenden Druckgasimpulsen zur Ausbildung von alternierenden Flüssigkeitsblöcken und Gasblöcken, die impulsartig entlang der Spülstrecke von der Einspeisestelle durch die Rohrleitung zur Ausspeisestelle getrieben werden. Die Vorrichtung umfasst wenigstens ein Kopplungselement zur Ankopplung des wenigstens einen zu reinigenden Hohlkanals an eine Druckimpulseinrichtung, mit der modulierende Druckgasimpulse erzeugt werden, die den zu reinigenden Hohlkanal mit modulierenden Druckgasimpulsen zur Ausbildung von alternierenden Flüssigkeitsblöcken und Gasblöcken beaufschlagt, die impulsartig entlang einer Spülstrecke von einer Einspeisestelle durch den Hohlkanal zur Entfernung von Ablagerungen an den Wänden des Hohlkanals zu einer Ausspeisestelle getrieben werden. Der vor der Spülstrecke angeordnete Vorlaufkanal für die Flüssigkeitsblöcke bewirkt, dass sich die Flüssigkeitsblöcke bis zum Anfang der Spülstrecke des Hohlkanals des medizinischen Instruments leitungsausfüllend ausbilden können.

Zur erfindungsgemäßen Beschleunigung des Flüssigkeitsvolumens in der Spülstrecke umfasst die Vorrichtung wenigstens einen Vorlaufkanal, der sich vor der Spülstrecke des Hohlkanals des medizinischen Instruments befindet. Jeder Vorlaufkanal ist vor der Beaufschlagung mit den Druckgasimpulsen mit einer Spülflüssigkeit teilbefüllbar und hinsichtlich seiner Leitungsgeometrie, seines Leitungsdurchmessers und/oder seiner Leitungslänge so dimensioniert, dass sich bei Beaufschlagung des Druckgasgemisches die Flüssigkeitsblöcke innerhalb des Vorlaufkanals vollständig ausbilden können, um den Leitungsquerschnitt der sich daran anschließenden Spülstrecke leitungsausfüllend zu durchwandern.

Die Vorrichtung ist vorzugsweise mit einem Barcodescanner oder RF-ID Scanner zur Registrierung von Nutzer und/oder medizinischem Instrument (z.B. Endoskop) ausgestattet. Dies ermöglicht, dass registriert werden kann, welcher Benutzer die Reinigung durchführt oder welches medizinische Gerät gereinigt wird. Dadurch erfolgt einerseits eine genaue Dokumentation und andererseits kann ein für ein bestimmtes Instrument eingestelltes Programm automatisch ablaufen. In einer Ausführungsvariante verfügt die Vorrichtung über einen Touchscreen. Über den Screen wird der Anwender durch alle Prozessschritte geleitet und bekommt Feedback. Das medizinische Instrument ist mit einem Kopplungselement an das Gerät angeschlossen. Ein Leuchtring um den Anschlusspunkt gibt dabei farbliches Feedback. Unterhalb befindet sich ein Tank, in dem Reinigungsflüssigkeit bevorratet ist.

In einer weiteren bevorzugten Ausführungsvariante umfasst die Vorrichtung Sensoren. Hiermit können Reinigungsparameter wie vorzugsweise Druck, Trübung, elektrischer Leitfähigkeit oder optischer Durchlässigkeit gemessen werden. Drucksensoren können darüber hinaus zur Dichtheitsprüfung dienen. Dies kann nach der Vorreinigung erfolgen, indem der Druckverlauf in der geschlossenen Vorrichtung bei einem vorgegebenen Druck gemessen wird.

Das oben beschriebene Verfahren läuft bevorzugt vollautomatisiert ab und ermöglicht einen validierbaren Ablauf einschließlich Vorreinigung, Desinfektion und Konditionierung. Daher verfügt die Vorrichtung in einer solchen Ausführungsform vorzugsweise über eine Regeleinrichtung, mit der Prozessparameter wie Druck, Durchflussmenge, Intervalldauer, Intervalllänge automatisiert erfasst und angepasst werden.

Eine solche Vorrichtung zur Vorreinigung von medizinischen Instrumenten mittels Impulsspülverfahren steht häufig auf Spültischen. Sie lässt sich aber auch stationär in Desinfektionsgeräte integrieren. Weiterhin ist es aber auch möglich, mobile Geräte mit dieser Technik auszurüsten. Diese Möglichkeit erlaubt es, beispielsweise in Katastrophenfällen, medizinische Geräte vor Ort instand zu halten, anstatt sie zeitaufwändig zu bestehenden Reinigungseinrichtungen zu bringen.

Das erfindungsgemäße Verfahren und die Vorrichtung ermöglichen einerseits die Prozessparameter wie Reinigungsdauer, Zulauf des Wassers und des Druckgases, ggf. weiterer Hilfsstoffe automatisch zu regeln und andererseits den Zustand der zu reinigenden medizinischen Instrumente bereits vor dem Entnehmen zu kontrollieren.

Das erfindungsgemäße Verfahren und die Vorrichtung bieten somit eine erhöhte Sicherheit für Anwender und Patienten und sind wesentlich ressourcenschonender, indem Reinigungsflüssigkeiten und medizinische Einmalprodukte eingespart werden.

Die Erfindung wird in den nachfolgenden Zeichnungen näher erläutert. Keinesfalls ist die Erfindung auf die hier gezeigten Ausführungsformen beschränkt. Auch die Kombination einzelner Ausführungsvarianten, einzelner Merkmale oder Merkmalskombinationen ist von der Erfindung umfasst.

### Beschreibung der Abbildungen

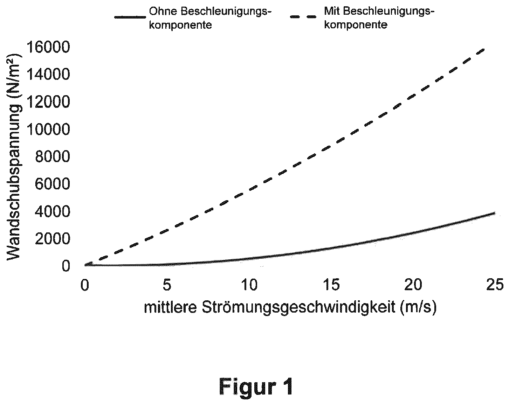

In Fig. 1 ist ein Vergleich des erfindungsgemäßen Impulsspülverfahrens mit Beschleunigungskomponente (d.h. mit Vorlaufkanal) und ohne Beschleunigungskomponente (Anschluss direkt an Spülstrecke) gezeigt. Dargestellt ist die Wandschubspannung der Spülstrecke im Verhältnis zur mittleren Strömungsgeschwindigkeit. Die Fließgeschwindigkeit der Wasserblöcke liegt bei einer Impulsspülung im Bereich zwischen 15 und 20 m/s. Durch die Anordnung eines Vorlaufkanals vor der Spülstrecke wird eine Beschleunigungsstrecke geschaffen, so dass die daraus resultierende Beschleunigungskomponente die Wandschubspannung signifikant erhöht.

Ein entscheidender Vorteil der automatisierten Reinigung ist die deutliche Ersparnis beim Wasserbedarf von bis zu 95 %. Der geringere Wasserbedarf und die Reinigungsdauer verringern dadurch den Ressourcenbedarf und damit die Gesamtkosten erheblich.

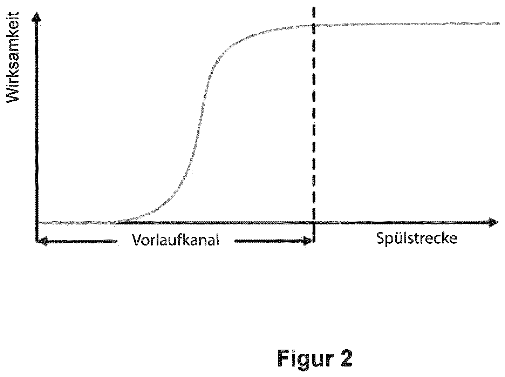

In Fig. 2 ist erkennbar, welchen Einfluss der Vorlaufkanal auf den Reinigungserfolg hat. Die Wirksamkeit der Reinigung baut sich im Verlauf des Vorlaufkanals auf und ist erst zu Beginn der Spülstrecke vollständig ausgebildet. Durch den vor der Spülstrecke angeordnete Vorlaufkanal wird die Wirksamkeit der Reinigung im Vergleich zur herkömmlichen Impulsspülung ohne Vorlaufkanal über den gesamten Reinigungsabschnitt nochmals deutlich gesteigert, da eine kontrollierte Beschleunigung und Aufbau der Flüssigkeitsblöcke im Vorlaufkanal möglich sind.

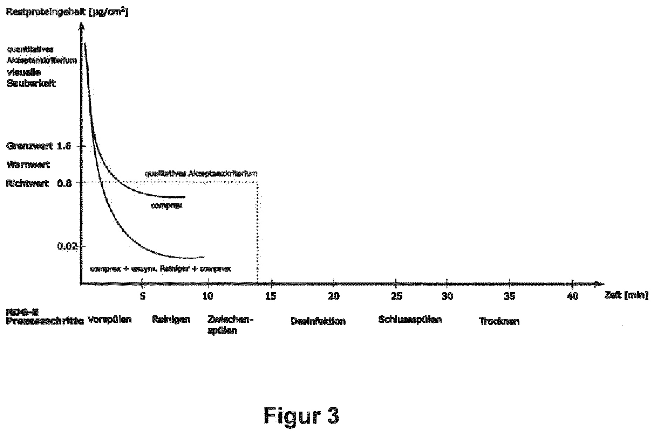

In Fig. 3 ist eine schematische Darstellung eines Vergleichs der Reinigungsleistung durch das erfindungsgemäße Impulsspülverfahren allein und dem erfindungsgemäßen Impulsspülverfahren in Kombination mit einem enzymatischen Reiniger gezeigt. Gemessen wurde der Restproteingehalt in µg/cm² in einem Hohlkanal des medizinischen Instruments in Abhängigkeit von der Zeit in Minuten. Die Prozessschritte umfassen: Vorspülen, Reinigen., Zwischenspülen, Desinfektion, Schlussspülen und Trocknen. Das quantitative Akzeptanzkriterium ist so definiert, dass ein Richtwert der Reinigungsleistung von 0,8 Restproteingehalt in µg/cm² innerhalb von 14 Minuten erreicht wird (RDG-E, CEN/ISO, 2019. DIN EN ISO 15883-4:2019-06; Reinigungs-Desinfektionsgeräte - Teil 4: Anforderungen und Prüfverfahren für Reinigungs-Desinfektionsgeräte mit chemischer Desinfektion für thermolabile Endoskope). Durch die alleinige Anwendung des erfindungsgemäßen Impulsspülverfahrens wird der Richtwert von 0,8 µg/cm² bereits in weniger als 5 Minuten unterschritten. Bei der Anwendung des erfindungsgemäßen Impulsspülverfahrens mit einem enzymatischen Reiniger konnte innerhalb von 5 Minuten eine Restproteingehalt von nur 0,02 µg/cm² erreicht werden.

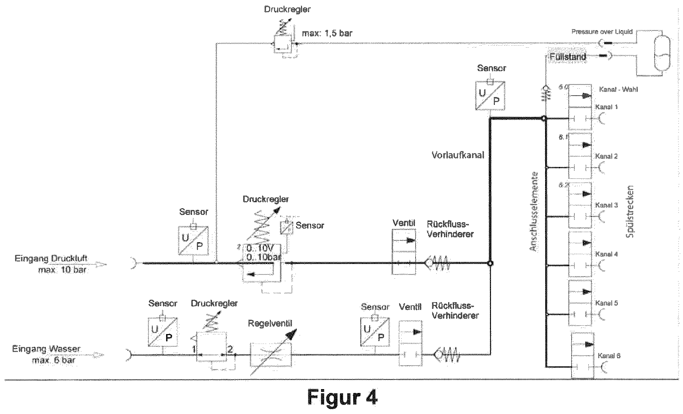

In Fig. 4 ist ein schematischer Aufbau der erfindungsgemäßen Vorrichtung und der Verfahrensablauf gezeigt. An einer Einspeisestelle wird dem Vorlaufkanal in der gezeigten Ausführungsvariante Wasser mit einem Druck von maximal 6 bar zugeführt. Zur Messung des Wasserdrucks ist ein Drucksensor eingebunden. Zur Regelung der Flüssigkeitszufuhr sind ein Druckregler und mehrere Ventile vorgesehen. Ein Rückflussverhinderer stellt sicher, dass das Wasser nicht durch das Ventil zurückgezogen werden kann. Die Regelungseinrichtungen und Sensoren kontrollieren die Teilbefüllung des Vorlaufkanals, die für die Durchführung des Impulsspülverfahrens essentiell ist.

Über einen getrennten Zugang wird Druckluft mit einem Druck von max. 10 bar zugeführt. Ein Ventil erzeugt modulierende Druckimpulse zum aktiven Vorlaufkanal. Die Flüssigkeit in dem teilbefüllten Vorlaufkanal wird beschleunigt. Es entstehen Gas- und Flüssigkeitsblöcke, die innerhalb des Vorlaufkanals noch weiter beschleunigt werden. Die Regelung des Druckes erfolgt ebenfalls über Ventile. Ein Rückflussverhinderer stellt sicher, dass die Druckluft nicht durch das Ventil zurückgelangen kann. Die in den Vorlaufkanal beaufschlagte Druckluft und das Wasser vermengen sich nur minimal im Lumen des Vorlaufkanals. Ein Sensor am Vorlaufkanal misst den herrschenden Druck. Die sich anschließenden Spülstrecken (d.h. Hohlkanäle 1 bis 6) des zu reinigenden medizinischen Instruments werden von den durch den Hohlkanal durchlaufenden alternierenden Gas- und Flüssigkeitsblöcken gereinigt.

Der Figur 4 ist ferner zu entnehmen, dass ein Teil der Druckluft in einer separaten Zuführleitung mit einem Druckregler auf einen Druck von maximal 1,5 bar reguliert wird. In einem Behälter befinden sich z.B. Hilfsstoffe, Desinfektionsmittel, Enzyme oder Chemikalien. Diese werden bevorzugt mittels Druckluft über eine separate Zuführleitung in den Vorlaufkanal gepresst. Ein Rückflussverhinderer verhindert, dass das Gemisch durch die beim Impulsspülverfahren erzeugten Druckblasen in den Behälter zurückgedrückt wird.

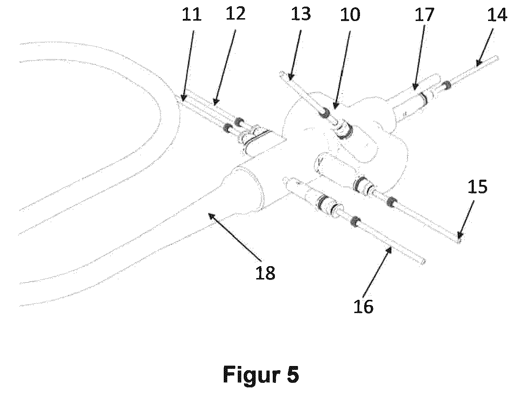

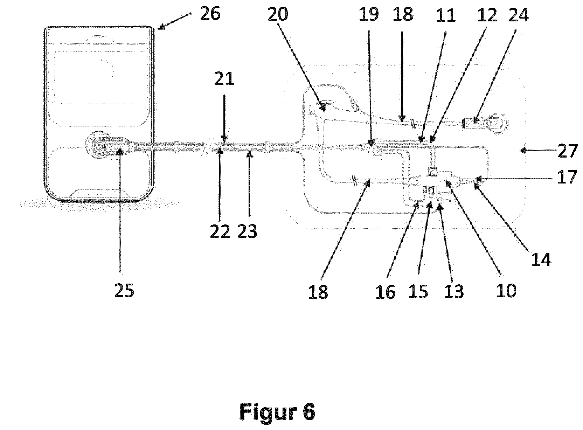

In Fig. 5 sind mehrere Leitungen 11, 12, 13, 14, 15, 16 mit Anschlusselementen zur Verbindung der Vorrichtung an ein medizinisches Instrument, d.h. in diesem Fall ein Endoskop, gezeigt. Die Verbindung der einzelnen Leitungen 11, 12, 13, 14, 15, 16 erfolgt über einen Versorgungsstecker 10. Die Leitungen 11, 12, 14, 15, 16 mit ihren Anschlusselementen werden an die vorhandenen zu reinigenden Hohlkanäle angeschlossen und gereinigt. Über diese Leitungen 11, 12, 14, 15, 16 und ihren Anschlusselementen wird die Druckimpulseinrichtung 26 mit dem Versorgungsstecker 10 des medizinischen Instruments verbunden (vgl. Fig. 6). Mittels der von der Druckimpulseinrichtung 26 erzeugten Druckluftimpulse lassen sich die Anschlüsse und Hohlkanäle im Versorgungsschlauch 18 des medizinischen Instruments reinigen. Da das Verfahren und die Vorrichtung für die Reinigung verschiedener medizinischer Instrumente von unterschiedlichen Herstellern mit wenigstens einem Hohlkanal geeignet ist, ist eine Vielzahl verschiedener Anschlüsse vorgesehen, die mit der Vorrichtung verbunden werden können. Eine zusätzlich vorhandene Farbkodierung ermöglicht eine klare Zuordnung der einzelnen Leitungen. Die Leitung 13 mit ihrem Anschlusselement dient der Verbindung der Druckimpulseinrichtung 26 mit der Leitung 3 für den Dichtigkeitstest. Am Versorgungsstecker 10 ist ferner ein Optik-Anschluss 17 für das Endoskop vorgesehen.

In Fig. 6 ist ein schematischer Aufbau der erfindungsgemäßen Vorrichtung bei einer Endoskopaufbereitung gezeigt. Die Druckerzeugungseinrichtung 26 ist mittels Kopplungselement 25 und Leitungen 11, 12, 13, 14, 15, 16 mit Anschlusselementen an ein medizinisches Instrument gekoppelt. Das medizinische Instrument besteht aus einem Bedienteil 20 für das Endoskop, einem Versorgungsschlauch 18 und einem Versorgungsstecker 10. Die Druckimpulseinrichtung 26 wird über entsprechende Anschlüsse und Zuleitungen an Luft, Wasser und Strom angeschlossen. Das medizinische Instrument ist über eine Leitung 21 und einem Leitungsbündel 22 mit der Druckimpulseinrichtung 26 über ein Kopplungselement 25 verbunden. In dem Leitungsbündel 22 werden die einzelnen Leitungen 11, 12, 14, 15, 16 geführt, die dann über einen Verteiler 19 in fünf Einzelleitungen aufgeteilt und einzeln mit dem Verbindungsstecker 10 verbunden werden.

Die Beaufschlagung mit modulierenden Druckimpulsen und die Teilbefüllung mit Spülflüssigkeit erfolgt wahlweise über eine oder mehrere aktive Leitungen. In der gezeigten Variante werden fünf Leitungen 11, 12, 14, 15, 16 in dem Leitungsbündel 22 geführt. Die Leitung 21 und die einzelnen Leitungen 11, 12, 14, 15, 16 bilden die einzelnen Vorlaufkanäle. Dabei umfasst jede einzelne mit dem Versorgungsstecker 10 verbundene Leitung 11, 12, 14, 15 und16 sowie die Leitung 21 einen separaten, von den anderen Leitungen unabhängigen Vorlaufkanal. Die Vorlaufkanäle beginnen bei der gezeigten Variante am geräteseitigen Leitungsanfang der Leitungen 11, 12, 14, 15, 16, 21. Die Leitungen 11, 12, 14, 15 und 16 enden am distalen Leitungsende am Versorgungsstecker 10, die Leitung 21 am Bedienteil des Endoskops. In den einzelnen Vorlaufkanälen findet die Beschleunigung der Flüssigkeitsblöcke mittels von der Druckimpulseinrichtung 26 erzeugten Druckimpulsen statt.

Die Vorrichtung ist geeignet, einen bestimmten Vorlaufkanal für die Reinigung aktiv zu schalten. Dazu wird der betreffende Vorlaufkanal, über den die Reinigung erfolgen soll, zunächst mit Flüssigkeit teilbefüllt. Anschließend erfolgt die Beaufschlagung der Druckimpulse von der Druckimpulseinrichtung 26. Am Ende des Vorlaufkanals der Leitungen 11, 12, 14, 15, 16, 21 beginnt die eigentliche Spülstrecke, d.h. spätestens in dem angeschlossenen Hohlkanal des medizinischen Instruments beginnt die Reinigung und hier ist die Reinigungseffizienz am größten, da dort die Flüssigkeitsblöcke und Druckluftimpulse am Stärksten ausgebildet sind. Die Effizienz der Reinigung ist letztendlich abhängig von den Vorlaufkanälen und der Teilbefüllung des Vorlaufkanals durch ein gezieltes Dosieren der Druckgaszufuhr, der Spülflüssigkeitszufuhr sowie der Impulsdauer und Intervalldauer.

Über das erfindungsgemäße Impulsspülverfahren lassen sich auch die einzelnen Anschlusselemente des Versorgungssteckers 10 oder des Bedienteils 20 des Endoskops reinigen. Die Spülstrecken beginnen spätestens bei den Anschlusselementen der Leitungen 11, 12, 14, 15, 16, 21 und umfassen alle in dem Versorgungsschlauch 18 liegenden Hohlkanäle bis zur Ausspeisestelle 24 am distalen Ende, die in das Reinigungsbecken 27 mündet. Die Leitung 13 mit Anschlusselement ist mit der Druckimpulseinrichtung 26 über das Kopplungselement 25 und den Kanal 23 für den Dichtigkeitstest verbunden.

Die Vorlaufkanäle werden an unterschiedlichen Positionen mit dem medizinischen Instrument verbunden, was von der Art und Anzahl der Hohlkanäle abhängt. Dadurch ist jede Anschlussstelle des medizinischen Instruments mit einer eigenen Druckimpulsleitung verbunden. Die Steuerung der einzelnen Spülstrecken erfolgt über eine Regeleinrichtung in der Druckimpulseinrichtung 26.

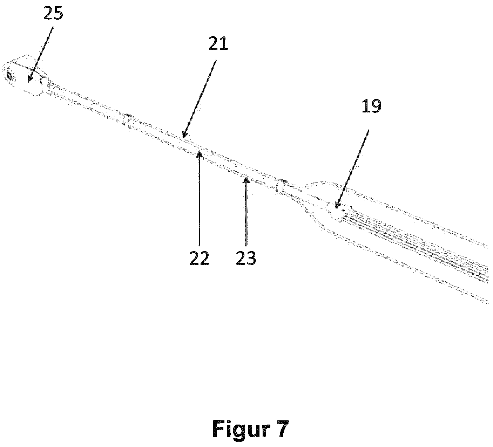

In Fig. 7 ist eine Variante eines erfindungsgemäßen Kopplungselement 25 gezeigt. Dieses wird an einer Seite an eine Druckimpulseinrichtung 26 angeschlossen und verbindet diese an der anderen Seite mit den zu reinigenden Hohlkanälen. Zu sehen sind die fünf in dem Leitungsbündel 22 geführten und aus dem Verteiler 19 hervorgehende Leitungen 11, 12, 14, 15, 16. Die Leitung 21 und die Leitungen 11, 12, 14, 15, 16 des Leitungsbündels 22, die von dem Verteiler 19 in Einzelleitungen aufgeteilt werden, umfassen die Vorlaufkanäle für die nachfolgenden Spülstrecken. Jeder Vorlaufkanal und jede Spülstrecke ist separat mit den Druckluftimpulsen beaufschlagbar und mit Spülflüssigkeit teilbefüllbar. Der teilbefüllte Vorlaufkanal dient dann als Beschleunigungsstrecke für die sich aufbauenden modulierenden Luft- und Flüssigkeitsblöcke.

Zusammengefasst ermöglicht das erfindungsgemäße Verfahren und die Vorrichtung eine automatisierte Reinigung von medizinischen Instrumenten mit wenigstens einem Hohlkanal, die schneller, hygienischer und mit gleichbleibender, messbarer Qualität durchgeführt wird.

### Bezugszeichenliste:

- 10: Versorgungsstecker
- 11: erste Leitung mit Anschlusselement
- 12: zweite Leitung mit Anschlusselement
- 13: Leitung mit Anschlusselement für Dichtigkeitstest
- 14: dritte Leitung mit Anschlusselement
- 15: vierte Leitung mit Anschlusselement
- 16: fünfte Leitung mit Anschlusselement
- 17: Optik-Anschluss
- 18: Versorgungsschlauch
- 19: Verteiler der Einzelkanäle nach dem Leitungsbündel
- 20: Bedienteil des Endoskops
- 21: Leitung mit Vorlaufkanal
- 22: Leitungsbündel bestehend aus den Einzelleitungen 11, 12, 14, 15, 16
- 23: Kanal für Dichtigkeitstest
- 24: Ausspeisestelle am distalen Ende
- 25: Kopplungselement
- 26: Druckimpulseinrichtung
- 27: Reinigungsbecken

## Patentansprüche

1. Verfahren zur Reinigung von medizinischen Instrumenten, die wenigstens einen zu reinigenden Hohlkanal umfassen, mittels modulierender Druckgasimpulse, umfassend:
a. Ankopplung des zu reinigenden Hohlkanals über ein oder mehrere Kopplungselemente an eine Druckimpulseinrichtung,
b. Beaufschlagung des Hohlkanals mit von der Druckimpulseinrichtung erzeugten modulierenden Druckgasimpulsen zur Ausbildung von alternierenden Flüssigkeitsblöcken und Gasblöcken, die impulsartig entlang einer Spülstrecke von einer Einspeisestelle durch den Hohlkanal zur Entfernung von Ablagerungen an den Wänden des Hohlkanals zu einer Ausspeisestelle getrieben werden,
**dadurch gekennzeichnet, dass** vor der Spülstrecke des Hohlkanals des medizinischen Instruments wenigstens ein Vorlaufkanal zur Beschleunigung des Flüssigkeitsvolumens in der Spülstrecke angeordnet wird, wobei der wenigstens eine Vorlaufkanal vor der Beaufschlagung mit den Druckgasimpulsen mit einer Spülflüssigkeit teilbefüllt wird, wobei der wenigstens eine Vorlaufkanal hinsichtlich seiner Leitungsgeometrie, seines Leitungsdurchmessers und/oder seiner Leitungslänge so dimensioniert wird, dass sich bei Beaufschlagung des Druckgasgemisches die Flüssigkeitsblöcke innerhalb des Vorlaufkanals vollständig ausbilden, um den Leitungsquerschnitt der sich daran anschließenden Spülstrecke leitungsausfüllend zu durchwandern, wobei die Teilbefüllung des Vorlaufkanals durch ein gezieltes Dosieren der Druckgaszufuhr, der Spülflüssigkeitszufuhr sowie der Impulsdauer und Intervalldauer erfolgt und die Teilbefüllung des Vorlaufkanals über Regelungseinrichtungen und Sensoren kontrolliert wird.

2. Verfahren nach Anspruch 1, **dadurch gekennzeichnet, dass** vor der Beaufschlagung des Hohlkanals mit modulierenden Druckgasimpulsen ein Drucktest zur Prüfung der Dichtigkeit des Raums zwischen Endoskophülle und der Außenseite der innenliegenden Kanäle des medizinischen Instruments durchgeführt wird.

3. Verfahren nach Anspruch 1, **dadurch gekennzeichnet, dass** vor der Beaufschlagung des Hohlkanals mit modulierenden Druckgasimpulsen ein Durchgängigkeitstest zur Prüfung des Verschleißes des Hohlkanals des medizinischen Instruments durchgeführt wird.

4. Verfahren nach einem der Ansprüche 1 bis 3, **dadurch gekennzeichnet, dass** für die Beaufschlagung des Hohlkanals mit modulierenden Druckgasimpulsen ein Vorratsgefäß für die Spülflüssigkeit bereitgestellt wird, bei dem ein definierter Vordruck eingestellt wird.

5. Verfahren nach einem der vorhergehenden Ansprüche, **dadurch gekennzeichnet, dass** vor oder zwischen der Beaufschlagung des Hohlkanals mit modulierenden Druckgasimpulsen ein Einwirkintervall vorgesehen ist, bei dem eine Spülflüssigkeit bereitgestellt wird, bei der bedarfsweise Hilfsstoffe, Desinfektionsmittel, Enzyme oder Chemikalien beigemengt wurden und die Spülflüssigkeit in dem zu reinigenden Hohlkanal für eine festgelegte Zeitdauer inkubiert wird.

6. Verfahren nach einem der vorhergehenden Ansprüche, **dadurch gekennzeichnet, dass** eine Beimengung von Hilfsstoffen, Desinfektionsmittel, Enzyme oder Chemikalien über eine separate Zuführleitung in den Vorlaufkanal erfolgt.

7. Verfahren nach Anspruch 6, **dadurch gekennzeichnet, dass** es sich bei dem Hilfsstoff um einen Stoff zur analytischen Bestimmung von Restkontaminationen handelt.

8. Verfahren nach einem der vorhergehenden Ansprüche, **dadurch gekennzeichnet, dass** der wenigstens eine Hohlkanal des medizinischen Instruments nach der Reinigung mit Druckluft getrocknet wird.

9. Vorrichtung zur Reinigung von medizinischen Instrumenten, die wenigstens einen zu reinigenden Hohlkanal umfassen, mittels Beaufschlagung des Hohlkanals modulierenden Druckgasimpulsen, umfassend:
a. wenigstens ein Kopplungselement (25) zur Ankopplung des wenigstens einen zu reinigenden Hohlkanals an eine Druckimpulseinrichtung (26),
b. eine Druckimpulseinrichtung (26), mit der modulierende Druckgasimpulse erzeugt werden, die den zu reinigenden Hohlkanal mit modulierenden Druckgasimpulsen zur Ausbildung von alternierenden Flüssigkeitsblöcken und Gasblöcken beaufschlagt, die impulsartig entlang einer Spülstrecke von einer Einspeisestelle durch den Hohlkanal zur Entfernung von Ablagerungen an den Wänden des Hohlkanals zu einer Ausspeisestelle (24) getrieben werden
**dadurch gekennzeichnet, dass** sich vor der Spülstrecke des Hohlkanals des medizinischen Instruments wenigstens ein Vorlaufkanal zur Beschleunigung des Flüssigkeitsvolumens in der Spülstrecke befindet, wobei der wenigstens eine Vorlaufkanal vor der Beaufschlagung mit den Druckgasimpulsen mit einer Spülflüssigkeit teilbefüllbar ist, wobei der wenigstens eine Vorlaufkanal hinsichtlich seiner Leitungsgeometrie, seines Leitungsdurchmessers und/oder seiner Leitungslänge so dimensioniert ist, dass sich bei Beaufschlagung des Druckgasgemisches die Flüssigkeitsblöcke innerhalb des Vorlaufkanals vollständig ausbilden können, um den Leitungsquerschnitt der sich daran anschließenden Spülstrecke leitungsausfüllend zu durchwandern, wobei Regelungseinrichtungen und Sensoren zur Kontrolle der Teilbefüllung des Vorlaufkanals vorgesehen sind und die Teilbefüllung durch ein gezieltes Dosieren der Druckgaszufuhr, der Spülflüssigkeitszufuhr sowie der Impulsdauer erfolgt.

10. Vorrichtung nach Anspruch 9, **dadurch gekennzeichnet, dass** sie einen Barcodescanner oder RF-ID Scanner zur Registrierung von Nutzer und/oder Endoskop umfasst.

11. Vorrichtung nach Anspruch 9 oder Anspruch 10, **dadurch gekennzeichnet, dass** sie Sensoren zur Messung von Reinigungsparametern, insbesondere Druck, Trübung, elektrischer Leitfähigkeit oder optischer Durchlässigkeit umfasst.

12. Vorrichtung nach einem der Ansprüche 9 bis 11, **dadurch gekennzeichnet, dass** eine Regeleinrichtung vorgesehen ist, mit der Prozessparameter wie Druck, Durchflussmenge, Intervalldauer, Intervalllänge automatisiert erfasst und angepasst werden.

13. Vorrichtung nach einem der Ansprüche 9 bis 12, **dadurch gekennzeichnet, dass** die zu reinigende Hohlkanäle des medizinischen Instruments einen Durchmesser von 0.5 bis 20 mm, vorzugsweise einen Durchmesser von 1 bis 7 mm aufweisen.

14. Vorrichtung nach einem der Ansprüche 9 bis 13, **dadurch gekennzeichnet, dass** es sich bei dem medizinischen Instrument um ein Endoskop, ein Gastroskop, ein Koloskop, ein Rektoskop, ein Proktoskop, ein Laparoskop, ein Arthroskop, ein Bronchoskop, ein Thoraskop, eine Sonde, ein Röhrchen, einen Schlauch, einen Katheter sowie stationäre Geräte handelt.

15. Vorrichtung nach einem der Ansprüche 9 bis 14, **dadurch gekennzeichnet, dass** mehrere Leitungen (11, 12, 13, 14, 15, 16) mit Anschlusselementen zur Verbindung der Vorrichtung an das medizinische Instrument vorgesehen sind, die an die vorhandenen zu reinigenden Hohlkanäle angeschlossen sind.

## Claims

1. A method for cleaning medical instruments comprising at least one hollow channel to be cleaned by means of modulating pressurized gas impulses, comprising:
a. coupling of the hollow channel to be cleaned via one or more coupling elements to a pressure impulse device,
b. impinging modulating pressurized gas impulses generated by the pressure impulse device to the hollow channel to form alternating liquid blocks and gas blocks that are driven in pulses along a flushing section from a feeding point through the hollow channel to a discharging point in order to remove sediments on the walls of the hollow channel,
**characterized in that** at least one pre-run channel for accelerating the volume of the liquid in the flushing section is provided upstream of the flushing section of the hollow channel of the medical instrument, wherein the at least one pre-run channel is partially filled with a flushing liquid before being impinged with the pressurized gas impulses, wherein in regard to its pipe geometry, its pipe diameter and/or its pipe length, the pre-run channel is dimensioned in way that, when impinging the pressurized gas mixture, the liquid blocks can completely form within the pre-run channel in order to travel through the pipe cross-section of the subsequent flushing section in a pipe-filling manner, wherein the partial filling of the pre-run channel takes place by specifically metering the pressurized gas supply, the flushing liquid supply as well as the impulse duration and interval duration and the partial filling of the pre-run channel is controlled by control devices and sensors.

2. The method according to claim 1, **characterized in that** a pressure test is performed before impinging modulating pressurized gas impulses to the hollow channel in order to check the tightness of the space between the endoscope sheath and the outside of the channels lying inside the medical instruments.

3. The method according to claim 1, **characterized in that** a consistency test is performed before impinging modulating pressurized gas impulses to the hollow channel in order to check the wear of the hollow channel of the medical instrument.

4. The method according to one of the claims 1 to 3, **characterized in that** a storage vessel for the flushing liquid is provided for impinging modulating pressurized gas impulses to the hollow channel, in which a defined initial pressure is set.

5. The method according to one of the preceding claims, **characterized in that** before or between impinging modulating pressurized gas impulses to the hollow channel, an exposure interval is provided in which a flushing liquid is provided to which auxiliary substances, disinfectants, enzymes or chemicals have been added, as required, and in which the flushing liquid is incubated in the hollow channel to be cleaned for a determined period of time.

6. The method according to one of the preceding claims, **characterized in that** the auxiliary substances, disinfectants, enzymes or chemicals are added to the pre-run channel via a separate supply line.

7. The method according to claim 6, **characterized in that** the auxiliary substance is a substance for the analytical determination of residual contamination.

8. The method according to one of the preceding claims, **characterized in that** the at least one hollow channel of the medical instrument is dried with pressurized air after cleaning.

9. A device for cleaning medical instruments comprising at least one hollow channel to be cleaned by impinging modulating pressurized gas impulses to the hollow channel, comprising:
a. at least one coupling element (25) for coupling the at least one hollow channel to be cleaned to a pressure impulse device (26)
b. a pressure impulse device (26) used for generating modulating pressurized gas impulses that impinges modulating pressurized gas impulses to the hollow channel to be cleaned to form alternating liquid blocks and gas blocks that are driven in pulses along a flushing section from a feeding point through the hollow channel to a discharging point (24) in order to remove sediments on the walls of the hollow channel,
**characterized in that** at least one pre-run channel for accelerating the volume of the liquid in the flushing section is provided upstream of the flushing section of the hollow channel of the medical instrument, wherein the at least one pre-run channel can be partially filled with a flushing liquid before being impinged with pressurized gas impulses, wherein in regard to its pipe geometry, pipe diameter and/or pipe length, the at least one pre-run section is dimensioned in a way that, when impinging the pressurized gas mixture, liquid blocks can completely develop within the pre-run channel in order to travel through the subsequent flushing section in a pipe-filling manner, wherein control devices and sensors are provided to control the partial filling of the pre-run channel and the partial filling takes place by specifically metering the pressurized gas supply, the flushing liquid supply and the impulse duration.

10. The device according to claim 9, **characterized in that** it comprises a barcode scanner or RF-ID scanner for registering the user and/or endoscope.

11. The device according to claim 9 or claim 10, **characterized in that** it comprises sensors for measuring cleaning parameters, particularly pressure, turbidity, electrical conductivity or optical permeability.

12. The device according to one of the claims 9 to 11, **characterized in that** a control device is provided to automatically detect and adjust process parameters like pressure, flow rate, interval duration, interval length.

13. The device according to one of the claims 9 to 12, **characterized in that** the hollow channels to be cleaned of the medical instrument have a diameter of 0.5 to 20 mm, preferably a diameter of 1 to 7 mm.

14. The device according to one of claims 9 to 13, **characterized in that** the medical instrument is an endoscope, a gastroscope, a colonoscope, a rectoscope, a proctoscope, a laparoscope, an arthroscope, a bronchoscope, a thoracoscope, a probe, a tube, a hose, a catheter or stationary devices.

15. The device according to one of the claims 9 to 14, **characterized in that** several pipelines (11, 12, 13, 14, 15, 16) with connecting elements for connecting the device to the medical instrument are provided, which are connected to existing hollow channels to be cleaned.

## Revendications

1. Procédé de nettoyage, au moyen d'impulsions de gaz sous pression modulantes, d'instruments médicaux munis d'au moins un canal creux à nettoyer, consistant à :
a. accoupler le canal creux à nettoyer, par l'intermédiaire d'un ou de plusieurs éléments(s) d'accouplement, à un appareil à impulsions de pression,
b. solliciter ledit canal creux par des impulsions de gaz sous pression modulantes engendrées par ledit appareil à impulsions de pression aux fins de former, en alternance, des agglomérats de liquide et des agglomérats de gaz qui sont entraînés en mode impulsionnel en parcourant le canal creux, le long d'un trajet de rinçage, d'une zone d'entrée à une zone de sortie, en vue d'éliminer des dépôts sur les parois dudit canal creux,
**caractérisé par le fait qu'**au moins un canal d'alimentation, conçu pour accélérer le volume de liquide dans le trajet de rinçage, est implanté avant ledit trajet de rinçage du canal creux de l'instrument médical, sachant que ledit canal d'alimentation, à présence minimale, est partiellement empli d'un liquide de rinçage préalablement à la sollicitation par les impulsions de gaz sous pression, lequel canal d'alimentation à présence minimale est dimensionné, quant sa géométrie de conduit, à son diamètre de conduit et/ou à sa longueur de conduit, de façon telle que les agglomérats de liquide se forment en totalité à l'intérieur dudit canal d'alimentation, lors d'une sollicitation du mélange de gaz sous pression, afin de migrer à travers la section transversale de conduit du trajet de rinçage qui s'y raccorde, en comblant l'intégralité dudit conduit, l'emplissage partiel dudit canal d'alimentation étant effectué moyennant un dosage adéquat de la délivrance de gaz sous pression, de la délivrance de liquide de rinçage, ainsi que de la durée des impulsions et de la durée des intervalles, et ledit emplissage partiel du canal d'alimentation étant contrôlé par l'intermédiaire d'appareils régulateurs et de capteurs.

2. Procédé selon la revendication 1, **caractérisé par le fait qu'**un test de pression est opéré, préalablement à la sollicitation du canal creux par des impulsions de gaz sous pression modulantes, en vue de vérifier l'étanchéité de l'espace situé entre une gaine endoscopique et la face externe des canaux logés à l'intérieur de l'instrument médical.

3. Procédé selon la revendication 1, **caractérisé par le fait qu'**un test de perméabilité est opéré, préalablement à la sollicitation du canal creux par des impulsions de gaz sous pression modulantes, aux fins de vérifier l'usure du canal creux de l'instrument médical.

4. Procédé selon l'une des revendications 1 à 3, **caractérisé par le fait qu'**un récipient de réserve dédié au liquide de rinçage, dans lequel une pression préalablie bien définie est réglée, est apprêté en vue de la sollicitation du canal creux par des impulsions de gaz sous pression modulantes.

5. Procédé selon l'une des revendications précédentes, **caractérisé par le fait qu'**il est prévu, avant ou entre la sollicitation du canal creux par des impulsions de gaz sous pression modulantes, un intervalle à effet opérant durant lequel est tenu en attente un liquide de rinçage dans lequel des adjuvants, des agents de désinfection, des enzymes ou des produits chimiques ont été ajouté(e)s en fonction des besoins, et ledit liquide de rinçage est incubé dans le canal creux à nettoyer, pour une durée fermement établie.

6. Procédé selon l'une des revendications précédentes, **caractérisé par le fait qu'**un ajout d'adjuvants, d'agents de désinfection, d'enzymes ou de produits chimiques, dans le canal d'alimentation, est effectué par l'intermédiaire d'un conduit d'amenée distinct.

7. Procédé selon la revendication 6, **caractérisé par le fait que** l'adjuvant se présente comme une substance affectée à la détermination analytique de contaminations résiduelles.

8. Procédé selon l'une des revendications précédentes, **caractérisé par le fait que** le canal creux de l'instrument médical, à présence minimale, est séché par de l'air comprimé à l'issue du nettoyage.

9. Dispositif dévolu au nettoyage d'instruments médicaux munis d'au moins un canal creux à nettoyer, par sollicitation dudit canal creux au moyen d'impulsions de gaz sous pression modulantes, comprenant :
a. au moins un élément d'accouplement (25) destiné à accoupler le canal creux à nettoyer, à présence minimale, à un appareil (26) à impulsions de pression,
b. un appareil (26) à impulsions de pression par lequel sont engendrées des impulsions de gaz sous pression modulantes et qui sollicite ledit canal creux à nettoyer, par des impulsions de gaz sous pression modulantes, aux fins de former en alternance des agglomérats de liquide et des agglomérats de gaz qui sont entraînés en mode impulsionnel en parcourant le canal creux, le long d'un trajet de rinçage, d'une zone d'entrée à une zone de sortie (24), en vue d'éliminer des dépôts sur les parois dudit canal creux,
**caractérisé par le fait qu'**au moins un canal d'alimentation, conçu pour accélérer le volume de liquide dans le trajet de rinçage, est implanté avant ledit trajet de rinçage du canal creux de l'instrument médical, sachant que ledit canal d'alimentation, à présence minimale, peut être partiellement empli d'un liquide de rinçage préalablement à la sollicitation par les impulsions de gaz sous pression, lequel canal d'alimentation à présence minimale est dimensionné, quant sa géométrie de conduit, à son diamètre de conduit et/ou à sa longueur de conduit, de façon telle que les agglomérats de liquide puissent se former en totalité à l'intérieur dudit canal d'alimentation, lors d'une sollicitation du mélange de gaz sous pression, afin de migrer à travers la section transversale de conduit du trajet de rinçage qui s'y raccorde, en comblant l'intégralité dudit conduit, des appareils régulateurs et des capteurs étant prévus pour contrôler l'emplissage partiel dudit canal d'alimentation, et ledit emplissage partiel étant effectué moyennant un dosage adéquat de la délivrance de gaz sous pression, de la délivrance de liquide de rinçage, ainsi que de la durée des impulsions.

10. Dispositif selon la revendication 9, **caractérisé par le fait qu'**il inclut un scanneur de codes-barres ou un scanneur RFID, en vue de l'enregistrement d'un utilisateur et/ou d'un endoscope.

11. Dispositif selon la revendication 9 ou la revendication 10, **caractérisé par le fait qu'**il inclut des capteurs affectés à la mesure de paramètres de nettoyage, en particulier d'une pression, d'une turbidité, d'une conductivité électrique ou d'une transmittance optique.

12. Dispositif selon l'une des revendications 9 à 11, **caractérisé par le fait qu'**il est prévu un appareil régulateur par lequel des paramètres de processus tels qu'une pression, un débit, une durée d'intervalle, une longueur d'intervalle sont saisis et adaptés en mode automatisé.

13. Dispositif selon l'une des revendications 9 à 12, **caractérisé par le fait que** les canaux creux de l'instrument médical, devant être nettoyés, présentent un diamètre de 0,5 à 20 mm, de préférence un diamètre de 1 à 7 mm.

14. Dispositif selon l'une des revendications 9 à 13, **caractérisé par le fait que** l'instrument médical se présente comme un endoscope, un gastroscope, un coloscope, un rectoscope, un proctoscope, un laparoscope, un arthroscope, un bronchoscope, un thorascope, une sonde, une canule, un tuyau souple, un cathéter, ainsi que comme des appareillages fixes.

15. Dispositif selon l'une des revendications 9 à 14, **caractérisé par le fait que** plusieurs conduits (11, 12, 13, 14, 15, 16), dotés d'éléments de raccordement, sont prévus pour relier ledit dispositif à l'instrument médical et sont raccordés aux canaux creux présents, devant être nettoyés.
